# EUROPEAN PATENT APPLICATION

(11) **EP 3 115 465 A1**
(43) Date of publication of application: **11.01.2017**
(21) Application number: 15176290.3
(22) Date of filing: 10.07.2015
(51) Int. Cl.: C12Q 1/68

(54) **IMPROVED BIOMARKER SET FOR SALIVARY GLAND TUMOR DIAGNOSIS**

(71) Applicant: Nederlandse Organisatie voor toegepast- natuurwetenschappelijk onderzoek TNO, 2595 DA 's-Gravenhage (NL)
(72) Inventor: TSIVTSIVADZE, Evgeni, 2595 DA 's Gravenhage (NL); MATSE, Johannes, 2595 DA 's Gravenhage (NL); MONTIJN, Roy Christiaan, 2595 DA 's Gravenhage (NL); KEIJSER, Bart Jan Frederik, 2595 DA 's Gravenhage (NL)
(74) Representative: V.O.

(57) **Abstract**

The invention relates to a set of biomarkers for the diagnosis of salivary gland neoplasms wherein said set consists of at least four markers selected from the group of hsa-miR-449b, hsa-miR-374, hsa-miR-411, hsa-miR-599, hsa-miR-1285, hsa-miR-324-5p and hsa-miR-449a, a method for diagnosing the benign or malignant status of a salivary gland tumor and methods for screening for pharmaceuticals for the treatment of (malignant) salivary gland tumors.

## Description

The invention relates to the diagnosis of neoplasms, more particularly to the diagnosis of salivary gland neoplasms, especially with diagnosing the difference between benign and malignant salivary gland neoplastic lesions.

### INTRODUCTION

Salivary gland neoplasms comprise a diverse group of benign and malignant neoplastic lesions. Based on histopathological criteria, the World Health Organization distinguishes 37 subtypes, of which 13 are of benign status and 24 are of malignant status (1). Approximately 80% of all salivary gland neoplasms occur in the parotid gland (1). Clinical examination, ultrasound scanning (with or without fine needle aspiration cytology), preoperative CT-scan and MRI are the most commonly used diagnostic methods (1). Additional tools, such as salivary tests, may be helpful in assisting with clinical diagnosis of tumors in salivary glands.

Saliva is an easily accessible medium, containing a host of potential biomarkers (proteins, DNAs, RNAs) for detection of systemic and oral diseases. Particularly, biomolecules in saliva which have attracted much attention for diagnostic applications are microRNAs (miRNAs). MiRNAs are small non-coding RNAs consisting of 19-25 nucleotides that can inhibit translation by binding to complementary sequences in the 3' UTR of multiple target mRNAs, usually resulting in their silencing (2). Altered levels of miRNA expression have been implicated in the etiology of cancer (2,3) and have not only been investigated in tissue (4), serum (5), but also in saliva (6) for use in the diagnosis of cancer (7-11).

In a previous study, miRNAs expression in saliva was profiled from patients with benign and malignant parotid gland tumors, resulting in the identification of 6 potential biomarkers (mmu-miR-140-5p, hsa-let-7g, hsa-miR-15b, hsa-miR-132, hsa-miR-222, and hsa-miR-374) (6). A combination of four miRNAs (hsa-miR-15b, hsa-miR-132, hsa-miR-223 and mmu-miR-140-5p) could discriminate between patients with malignant and benign parotid gland tumors (sensitivity 69%; specificity 95%; AUC 0.9).

One of the potential limitations of the previous study, however, was the use of a univariate statistical approach to analyze these high-dimensional data. Univariate analysis involves the testing across conditions of one variable at a time. The tacit assumption is that the underlying biological process is dominated by a single process acting as an isolated entity. While such an approach is attractive due to its relative simplicity, biological processes such as tumor development are more complex by nature, driven by different multi-component interactions between, for example, DNA, RNA, proteins etc. Accordingly, the set of biomarkers that was found was sub-optimal and there remained a challenge to find a set of biomarkers with a higher sensitivity and/or specificity.

### SUMMARY OF THE INVENTION

The invention now resulted in a set of biomarkers that would give a better diagnosis for the classification of salivary gland neoplasms into benign and malignant. Therefore, the invention consists of a set of biomarkers for the diagnosis of salivary gland neoplasms where the set consists of at least four markers selected from the group of hsa-miR-449b, hsa-miR-374, hsa-miR-411, hsa-miR-599, hsa-miR-1285, hsa-miR-324-5p and hsa-miR-449a, preferably at least five of these biomarkers, more preferably at least six and most preferably at least seven. Further part of the invention is a kit for the diagnosis of such neoplasms harboring means to quantify the level of said set of biomarkers and further instructions to use the kit.
Also part of the invention is a method for the diagnosis of the status of salivary gland neoplasms comprising:
a) taking a saliva sample from a person;
b) determining the amount of any of the sets of biomarkers as defined above; and
c) on basis of these results classifying the salivary gland neoplasm as benign or malignant.

### LEGEND TO THE FIGURES

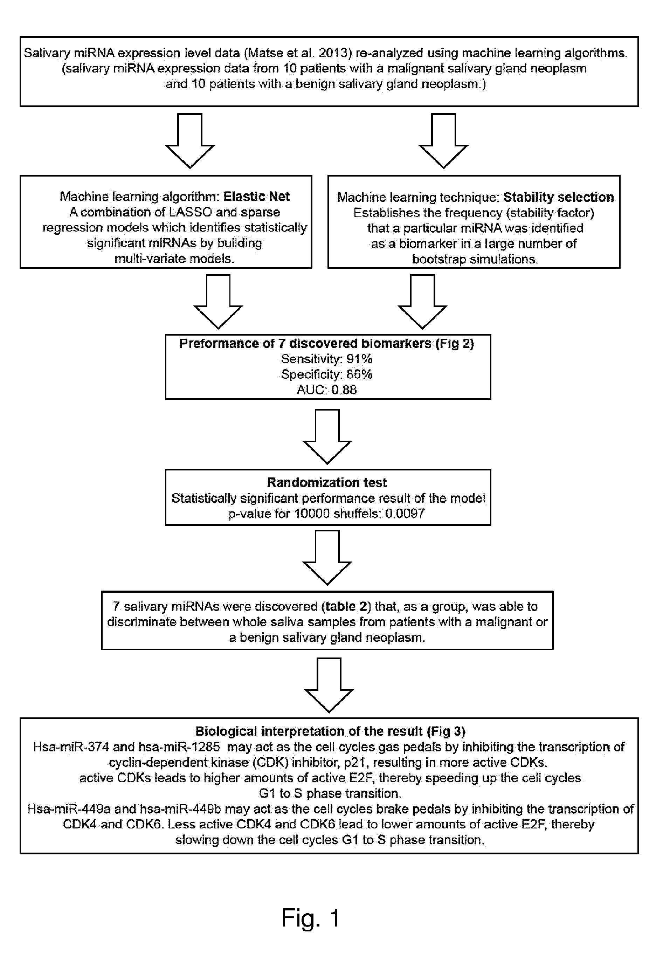

Figure 1. Overview of the research design and multivariate techniques used in the study.

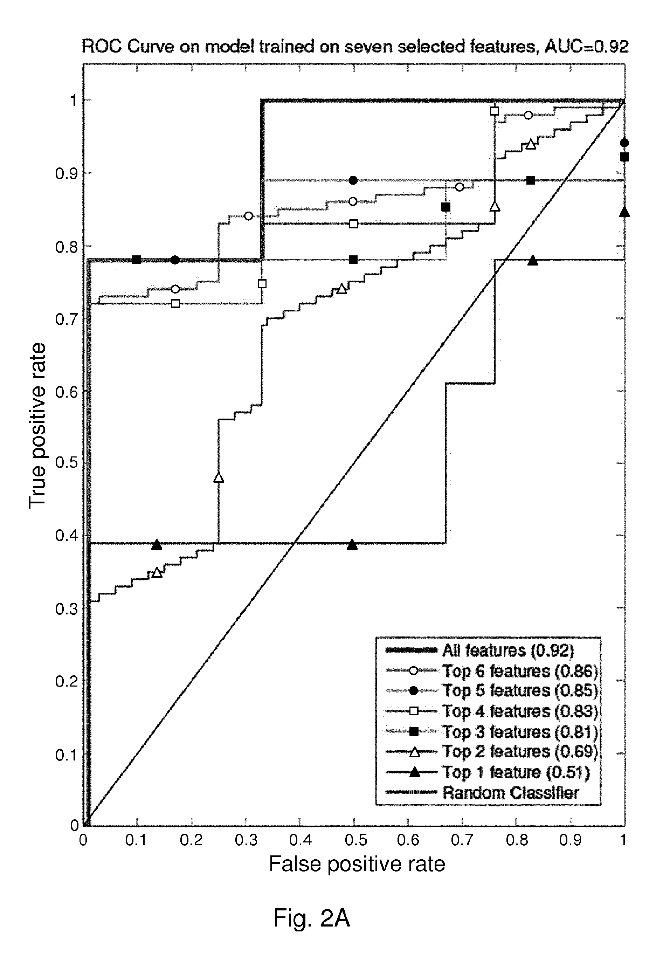

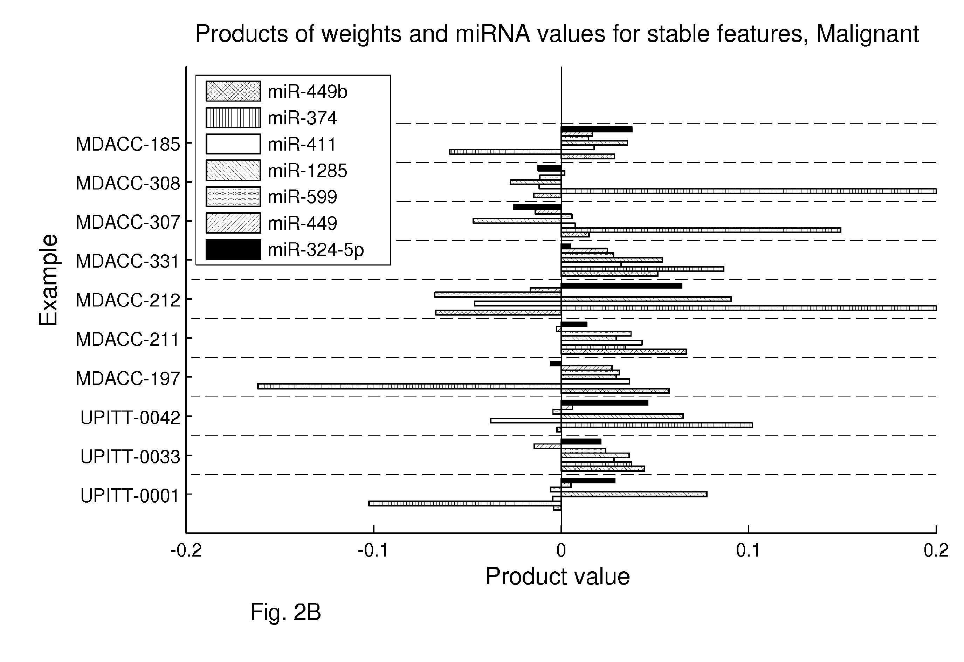

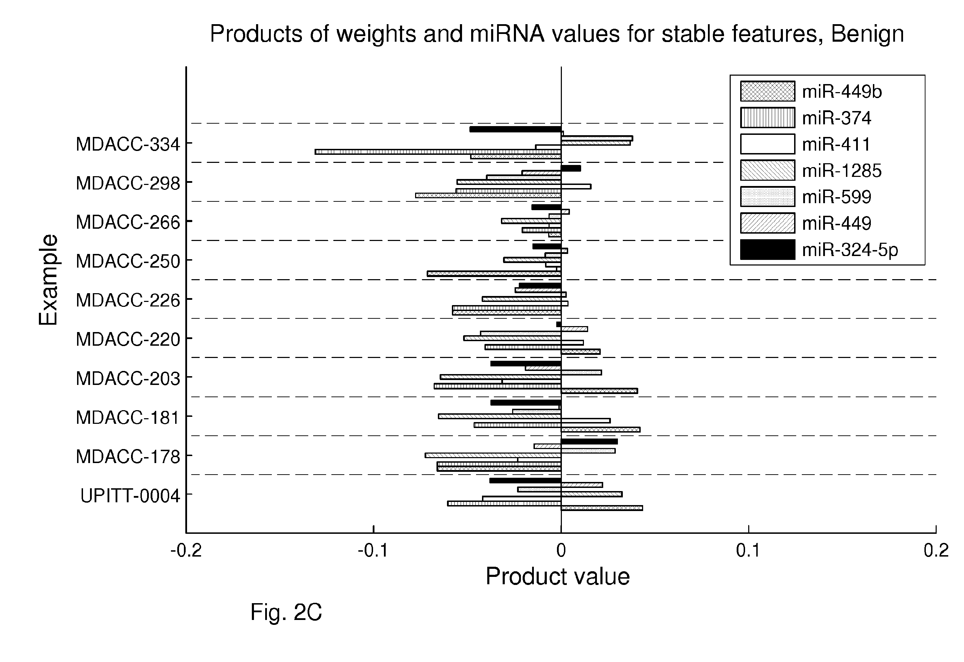

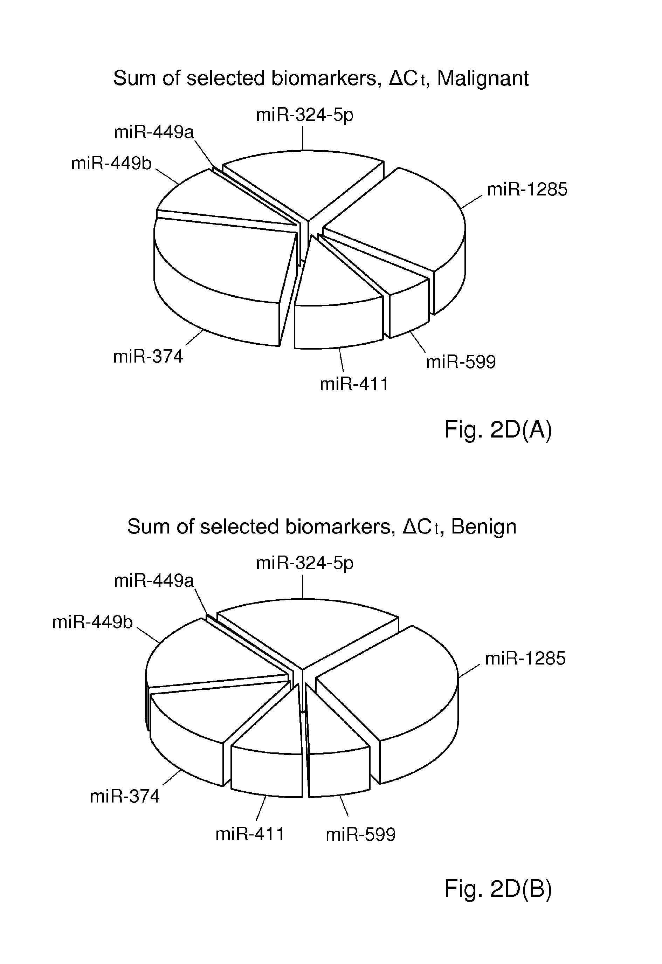

Figure 2. ROC curve and Bar chart of selected biomarkers' and weighting coefficients' products for malignant and benign tumor groups. A. Receiver-operating-characteristics (ROC) curve. ROC computed from final model. The best prediction model included 7 miRNAs, which in combination provided the best prediction. The area-under-the-curve value of the ROC curve was 0.92, the specificity was 86% and sensitivity was 91%. B. The product values for the malignant tumor samples. C. The product values for the benign tumor samples. The weights for the malignant samples go in the opposite direction in comparison to the weights of the benign samples. The width of the bar corresponds to the value of the product of weights and ΔCₜ. D. Piechart showing the "Sum of ΔCₜ of the selected 7 miRNA". A. pie-chart representing the distribution of the sum of ΔCₜ of the discovered miRNAs for malignant salivary gland tumors. B. pie-chart representing the distribution of the sum of ΔCₜ of the discovered miRNAs for benign salivary gland tumors

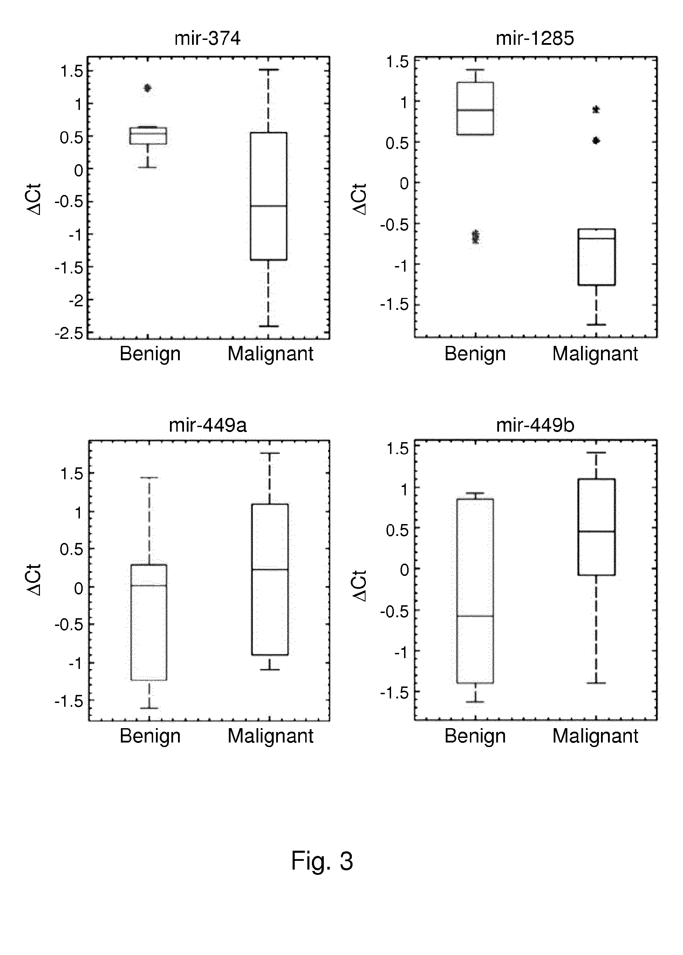

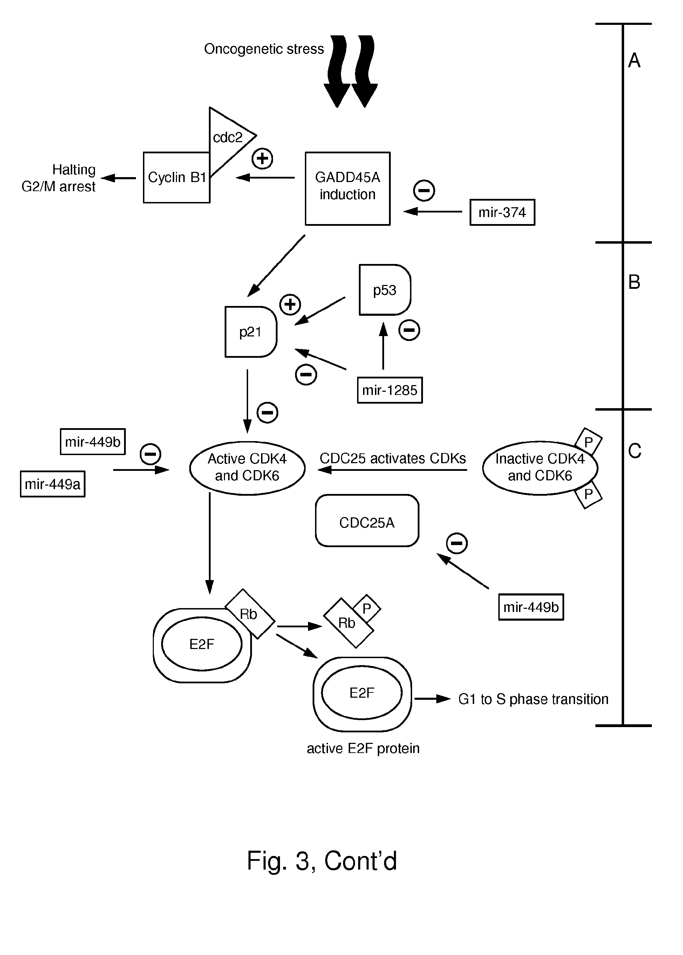

Figure 3. Biological interpretations of the validated targets. Hsa-miR-374 inhibits the transcription of growth arrest and DNA-damage-inducible protein (GADD45A) (Section A). GADD45A strengthens p21's Cyclin-dependent kinase (CDK) inhibitor activity and without it p21 is less capable of inactivating CDK4 and CDK6. Hsa-miR-1285 inhibits the transcription of p21 directly by binding p21 mRNA, but also indirectly by inhibiting the transcription of p21 activator, p53. By influencing these important processes, a higher expression of hsa-miR-374 and hsa-miR-1285 can speed up of the cell cycle (Section B). Hsa-miR-449a and hsa-miR-449b can inhibit the transcription of CDK4 and CDK6 (Section C) by directly binding the mRNA of CDK4 and CDK6 (Section C). Furthermore, hsa-miR-449b can also inhibit the transcription of CDK4 and CDK6 activator, cell division cycle 25A (CDC25A). By inhibiting the transcription of CDK4, CDK6 and CDC25A, by hsa-miR-449a and hsa-miR-449b, less active E2F is available, halting the cell cycles G1 to S phase transition. The expression levels of the miRNAs in saliva from patients with a malignant and benign salivary gland neoplasms are portrait on the left hand side. - inhibition; + activation.

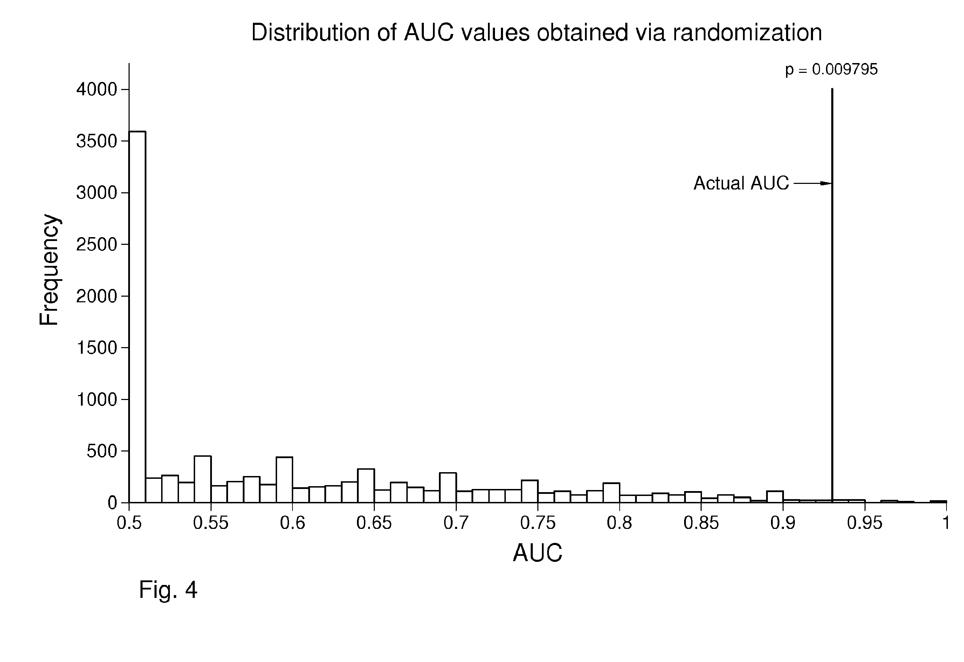

Figure 4. Histogram of AUC scores obtained via randomization test after 10000 shuffles.

### DETAILED DESCRTIPTION

The term "capable of specifically hybridizing to" refers to a nucleic acid sequence capable of specific base-pairing with a complementary nucleic acid sequence and binding thereto to form a nucleic acid duplex.
A "complement" or "complementary sequence" is a sequence of nucleotides which forms a hydrogen-bonded duplex with another sequence of nucleotides according to Watson-Crick base-paring rules. For example, the complementary base sequence for 5'-AAGGCT-3' is 3'-TTCCGA-5'.
The term "stringent hybridization conditions" refers to hybridization conditions that affect the stability of hybrids, e.g., temperature, salt concentration, pH, formamide concentration and the like. These conditions are empirically optimised to maximize specific binding and minimize nonspecific binding of the primer or the probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tm) for the specific sequence at a defined ionic strength and pH. The Tm is the temperature (under defined ionic strength and pH) at which 50% of a complementary target sequence hybridises to a perfectly matched probe or primer. Typically, stringent conditions will be those in which the salt concentration is less than about 1.0 M Na ion, typically about 0.01 to 1.0 M Na ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridization with a buffer solution of 30% formamide, 1 M NaCl, 1% SDS at 37°C and a wash in 2x SSC at 40°C. Exemplary high stringency conditions include hybridization in 50% formamide, 1 M NaCl, 1% SDS at 37°C, and a wash in 0.1x SSC at 60°C. Hybridization procedures are well known in the art and are described in e.g. Ausubel et al, Current Protocols in Molecular Biology, John Wiley & Sons Inc., 1994.
The term "oligonucleotide" refers to a short sequence of nucleotide monomers (usually 6 to 100 nucleotides) joined by phosphorous linkages (e.g., phosphodiester, alkyl and aryl-phosphate, phosphorothioate), or non-phosphorous linkages (e.g., peptide, sulfamate and others). An oligonucleotide may contain modified nucleotides having modified bases (e.g., 5-methyl cytosine) and modified sugar groups (e.g., 2'-O-methyl ribosyl, 2'-O-methoxyethyl ribosyl, 2'-fluoro ribosyl, 2'-amino ribosyl, and the like). Oligonucleotides may be naturally-occurring or synthetic molecules of double- and single-stranded DNA and double- and single-stranded RNA with circular, branched or linear shapes and optionally including domains capable of forming stable secondary structures (e.g., stem-and-loop and loop-stem-loop structures).

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primers will depend on many factors, including temperature and source of primer. A "pair of bi-directional primers" as used herein refers to one forward and one reverse primer as commonly used in the art of DNA amplification such as in polymerase chain reaction (PCR) amplification.

The term "probe" refers to a single-stranded oligonucleotide sequence that will recognize and form a hydrogen-bonded duplex with a complementary sequence in a target nucleic acid sequence analyte or its cDNA derivative.

"MicroRNAs" (miRNAs or MIRs) are short ribonucleic acid (RNA) molecules, on average only 22 nucleotides long and are found in nearly all eukaryotic cells. MIRs regulate gene activity by imperfect base pairing to the 3' UTR of their target mRNAs, leading to mRNA degradation or inhibition of translation. One single MIR may regulate expression of as many as 200 different gene targets, which means that expression of about one-third of human mRNAs might be controlled by MIRs. Altered expression of MIRs can therefore contribute to malignant transformation by increasing or decreasing expression of oncogenes and tumour suppressor genes, respectively (reviewed by Calin and Croce, Nat Rev Cancer 2006, 6, 857-866). The fact that expression profiling of a limited amount of MIRs provided a very accurate classifier of tumour subtypes, yielding even better results than a classifier based on genome-wide mRNA expression profiling, underlines the importance of miRNAs in carcinogenesis in general (Lu et al., Nature 2005;435:834-8). Interestingly, miRNA expression levels in tumours are globally lower compared to normal tissue. Like for protein encoding tumor suppressor genes, also silencing of miRNAs may results from epigenetic alterations, such as DNA methylation of regulatory sequences.

Most microRNA genes are found in intergenic regions or in antisense orientation to genes and contain their own miRNA gene promoter and regulatory units.

The invention provides a method for this diagnosis of salivary gland neoplasms comprising measuring the level of a plurality of miRNA biomarkers in test sample obtained from the subject, wherein said plurality of miRNA biomarkers comprises at least four miRNAs selected from the group consisting of hsa-miR-449b, hsa-miR-374, hsa-miR-411, hsa-miR-599, hsa-miR-1285, hsa-miR-324-5p and hsa-miR-449a, wherein an increased level of hsa-miR-449b, hsa-miR-599, and/or hsa-miR-449a and a reduced level of hsa-miR-324-5p, hsa-miR-1285, hsa-miR-411 and/or hsa-miR-374 relative to a normal control is indicative for the presence of a benign salivary gland neoplasm, while a decreased level of hsa-miR-449b, hsa-miR-599, and/or hsa-miR-449a and an increased level of hsa-miR-324-5p, hsa-miR-1285, hsa-miR-411 and/or hsa-miR-374 relative to a normal control is indicative for the presence of a malignant salivary gland neoplasm.
As is exemplified herein below, the expression of each of hsa-miR-324-5p, hsa-miR-1285, hsa-miR-411 and/or hsa-miR-374 was found to be significantly decreased (downregulated) in patients with benign parotid gland tumors, while in these patients the expression of the biomarkers hsa-miR-449b, hsa-miR-599, and/or hsa-miR-449a was increased. Accordingly, a reduced level of said miRNAs hsa-miR-324-5p, hsa-miR-1285, hsa-miR-411 and/or hsa-miR-374 and/or an increased level of said miRNAs hsa-miR-449b, hsa-miR-599, and/or hsa-miR-449a relative to a control indicates that the subject has a benign salivary gland neoplasm.
As is further exemplified herein below, the expression of each of hsa-miR-324-5p, hsa-miR-1285, hsa-miR-411 and/or hsa-miR-374 was found to be significantly increased (upregulated) in patients with malignant parotid gland tumors, while in these patients the expression of the biomarkers hsa-miR-449b, hsa-miR-599, and/or hsa-miR-449a was decreased. Accordingly, an increased level of said miRNAs hsa-miR-324-5p, hsa-miR-1285, hsa-miR-411 and/or hsa-miR-374 and/or a reduced level of said miRNAs hsa-miR-449b, hsa-miR-599, and/or hsa-miR-449a relative to a control indicates that the subject has a malignant salivary gland neoplasm.
In one aspect, a reduced level refers to an expression of the biomarker that is decreased by at least 2-fold, 3-fold, 4-fold, 5-fold, or 6-fold relative to control sample. In a preferred embodiment, there is at least a 10-fold reduction..

Exemplary combinations of miRNAs present in a set of biomarkers according to the invention include one or more of miR-499b, miR-374 and miR-411. In one aspect, a method of the invention comprises measuring the level of miR-499b, miR-374 and miR-411, with at least one additional marker selected from the group consisting of miR-599, miR-1285, miR-324-5p, and miR-449a. Specific exemplary panels include the following biomarker combinations:
(i) miR-449b, miR-374 and miR-411, with at least two additional marker selected from the group consisting of miR-599, miR-1285, miR-324-5p, and miR-449a.
(ii) miR-449b, miR-374, with at least two additional markers selected from the group consisting of miR-411, miR-599, miR-1285, miR-324-5p, and miR-449a.
(iii) miR-449b, miR-411, with at least two additional markers selected from the group consisting of miR-374, miR-599, miR-1285, miR-324-5p, and miR-449a.
(iv) miR-374, miR-411, with at least two additional markers selected from the group consisting of miR-499b, miR-599, miR-1285, miR-324-5p, and miR-449a.

Further exemplary combinations of miRNAs present in a set of biomarkers according to the invention include one or more of miR-599, miR-1285, and miR-324-5p. In one aspect, a method of the invention comprises measuring the level of miR-599, miR-1285, and miR-324-5p, with at least one additional marker selected from the group consisting of miR-449a, miR-449b, miR-374 and miR-411. Specific exemplary panels include the following biomarker combinations:
(v) miR-599, miR-1285, and miR-324-5p, with at least two additional markers selected from the group consisting of miR-449a, miR-449b, miR-374 and miR-411;
(vi) miR-599, miR-1285, and at least two additional markers selected from the group consisting of miR-324-5p, miR-449a, miR-449b, miR-374 and miR-411;
(vii) miR-599 and miR-324-5p, and at least two additional markers selected from the group consisting of miR-1285, miR-449a, miR-449b, miR-374 and miR-411;
(viii) miR-1285, miR-324-5p, and at least two additional markers selected from the group consisting of miR-599, miR-449a, miR-449b, miR-374 and miR-411.

It could further be advantageous to include other miRNAs, such as hsa-miR-519b-3p, hsa-miR-367 and/or hsa-miR-224 to further strengthen the diagnosis.

"miR-449b" as used herein indicates a mature miR-449b, i.e. a nucleic acid having at least 85% (e.g. at least 90%, 95%, 98%, or 100%) identity to the sequence CAGCCACAACUACCCUGCCACU (Sequence 1).

"miR-374" as used herein indicates a mature miR-374 i.e. a nucleic acid having at least 85% (e.g. at least 90%, 95%, 98%, or 100%) identity to the sequence UUAUAAUACAACCUGAUAAGUG (Sequence 2).

"miR-411" as used herein indicates a mature miR-411 i.e. a nucleic acid having at least 85% (e.g. at least 90%, 95%, 98%, or 100%) identity to the sequence UAGUAGACCGUAUAGCGUACG (Sequence 3).

"miR-599" as used herein indicates a mature miR-599 i.e. a nucleic acid having at least 85% (e.g. at least 90%, 95%, 98%, or 100%) identity to the sequence GUUGUGUCAGUUUAUCAAAC (Sequence 4).

"miR-1285" as used herein indicates a mature miR-1285 i.e. a nucleic acid having at least 85% (e.g. at least 90%, 95%, 98%, or 100%) identity to the sequence UCUGGGCAACAAAGUGAGACCU (Sequence 5).

"miR-324-5p" as used herein indicates a mature miR-324-5p i.e. a nucleic acid having at least 85% (e.g. at least 90%, 95%, 98%, or 100%) identity to the sequence CGCAUCCCCUAGGGCAUUGGUGU (Sequence 6).

"miR-449a" as used herein indicates a mature miR-499a i.e. a nucleic acid having at least 85% (e.g. at least 90%, 95%, 98%, or 100%) identity to the sequence UGGCAGUGUAUUGUUAGCUGGU (Sequence 7).
Whereas a biomarker panel of the invention can contain only at least four miRNA biomarkers, the skilled person will appreciate that the reliability generally increases with increasing the number of biomarkers. Accordingly, in one embodiment the measuring comprises measuring the level of at least five, preferably at least six, of said hsa-miR-449b, hsa-miR-374, hsa-miR-411, hsa-miR-599, hsa-miR-1285, hsa-miR-324-5p and hsa-miR-449a. In a specific aspect, the level of each of said hsa-miR-449b, hsa-miR-374, hsa-miR-411, hsa-miR-599, hsa-miR-1285, hsa-miR-324-5p and hsa-miR-449a is measured. Hence, also provided is a circulating miRNA profile for determining whether a salivary gland tumor is benign or malignant, comprising at least four, preferably at least five, more preferably at least six, most preferably all of said hsa-miR-449b, hsa-miR-374, hsa-miR-411, hsa-miR-599, hsa-miR-1285, hsa-miR-324-5p and hsa-miR-449a.

A method of the invention comprises measuring the level of a plurality of miRNAs in a test sample obtained (isolated) from the subject. The invention thus relates to a method of determining in *vitro* whether a subject has a benign or a malignant salivary gland tumor The subject is preferably a human subject.
"Test sample" is used in its broadest sense as containing nucleic acids. More specifically, any biological test sample comprising or suspected to comprise circulating miRNAs of the subject can be used. A sample may comprise a bodily fluid such as blood, saliva or urine. A preferred sample for detection is a saliva sample.
The amount of miRNAs associated with benign or malignant salivary gland tumors may be determined after isolation of RNA from a sample. Methods for isolating RNA are known in the art, including the use of commercial RNA isolation kits such as, for example, mirVana PARIS kit (Ambion), miRCURY RNA Isolation Kits - Biofluids (Exiqon) or Trizol LS (Invitrogen). The isolation of RNA is typically performed in the presence of a strong denaturant such as GITC, LiCl, SDS and/or phenol in order to inactivate RNase, if present. Alternatively, a biological sample can be processed for detection of miRNA sequences without prior isolation of RNA, for example by isolating vesicles such as microvesicles from a sample.
With regard to the determination of the panel of miRNAs of the invention, the expression of these miRNAs can be measured separately or simultaneously. In addition to determining the level of at least four biomarkers from the set of biomarkers described herein above, the method may comprise measuring additional miRNAs that can provide useful biological information, in particular relating to the presence and nature of tumors, more particularly salivary gland tumors. As shown herein below, the present inventors identified that the expression of each of hsa-miR-449b, hsa-miR-374, hsa-miR-411, hsa-miR-599, hsa-miR-1285, hsa-miR-324-5p and hsa-miR-449a was significantly changed between persons having a benign and person having a malignant salivary gland neoplasm.

In a previous study (6) the inventors identified 6 potential biomarkers (mmu-miR140-5p, hsa-let-7g, hsa-miR-15b, hsa-miR-132, hsa-miR-222, and hsa-miR-374) that were differently expressed in whole saliva from patients with benign parotid neoplasms and malignant parotid neoplasms. A combination of four miRNAs (hsa-miR-15b, hsa-miR-132, hsa-miR-223 and mmu-miR-140-5p) was shown to discriminate between patients with a malignant and a benign salivary gland tumor with a sensitivity of 69%, and a specificity of 95% (AUC 0.9). Unlike in said previous study, where univariate statistical techniques were used to analyze the miRNAs expression in whole saliva from patients with benign and malignant parotid gland tumors, in the present invention a range of machine learning techniques was used that uncovered a completely different panel of miRNAs comprising only two of the previous found miRNAs (hsa-miR-374 and hsa-miR-519-3p). The other miRNAs may have been missed in the previous study because, unlike the present invention, the previous study looked at fold-change and/or p-value alone, instead of miRNAs that have group-based correlations.
Key factors in the present analysis are the novel machine learning methods that allow to utilize the full potential of the gathered data. Specifically, the elastic net algorithm allows for building multivariate models, which can lead to the identification of a set of biomarkers that have a mechanistic effect in the tumor cell. The further used stability selection procedure is particularly well-suited for medium and small sized data collections where the number of patients diagnosed with a malignant form of disease is limited and the amount of variables, in this case miRNAs, is much larger than the sample size.

Of the seven selected miRNAs, four (hsa-miR-374, hsa-miR-449a, hsa-miR-449b and hsa-miR-1285) have been associated with validated targets (18-22 and 30-31), which could influence the speed of the cell cycle, while for the other three (hsa-miR-324-5p, hsa-miR-411 and hsa-miR-599) no targets have been validated yet. The expression levels of hsa-miR-449a, hsa-miR-449b and hsa-miR-599 were higher in whole saliva from patients with a benign salivary gland tumor, whereas the expression of hsa-miR-324-5p, hsa-miR-374, hsa-miR-411 and hsa-miR-1285 was higher in whole saliva of patients with a malignant salivary gland tumor.
Two (hsa-miR-374 and hsa-miR-1285) of the seven selected miRNAs have validated targets among which are p21 and the growth arrest and DNA-damage-inducible protein (GADD45A) (18, 19). By binding to their target mRNA hsa-miR-374 and hsa-miR-1285 can influence the transcription of p21 and GADD45A, which could result in a speeding up of the cell cycle (Fig. 3 section A and B). Hsa-miR-375 influences p21 indirectly by binding to the mRNA of GADD45A (Fig. 3 section A) (20, 21). GADD45A strengthens p21's Cyclin-dependent kinase (CDK) inhibitor activity and without it p21 is less capable of inactivating CDK4 and CDK6. Hsa-miR-1285 can influence p21 transcription directly by binding to its mRNA, or indirectly by binding to the mRNA of p21s activator, p53 (Fig. 3 section B) (20, 21). A higher expression of hsa-miR-374 and hsa-miR-1285 results in more active CDKs and higher amounts of active E2F, speeding up the G1 to S phase transition. Hsa-miR-374 and hsa-miR-1285, therefore, may act as the cell cycle's "gas-pedal". Two of the seven selected miRNA (hsa-miR-449a and hsa-miR-449b, have validated targets which could result in a slowing down of the cell cycle. hsa-miR-449a and hsa-miR-449b can inhibit the transcription of CDK4 and CDK6 (Fig. 3 section C) (19, 22, 23) by directly binding to their 3' UTR mRNA region. Furthermore, hsa-miR-449b also inhibits the transcription of cell division cycle 25A (CDC25A) (24). CDC25A activates CDK4 and CDK6 at a specific time in the cell cycle. A lower transcription of CDK4, CDK6 and CDC25A may result in less phosphorylated Rb/E2F complexes and lower amounts of active E2F, halting the G1 to S phase transition (22). A higher expression of hsa-miR-449a and hsa-miR-449b may lead to a halting of the cell growth and therefore, hsa-miR-449a and hsa-miR-449b can be seen as the "brake-pedal" of the cell cycle.
This is the first finding of a salivary miRNA biomarker panel that not only can distinguish saliva samples from patients with a benign and patients with a malignant salivary gland neoplasm, but also gives a hypothetical insight about the pathology of salivary gland neoplasm.

Quantification of the relevant miRNAs in a sample may be performed using any method known in the art for quantification of miRNA or other small RNAs. In one embodiment, the method comprises measuring the level of said miRNAs using RT-PCR, a biochip, quantitative PCR, serial analysis of gene expression (SAGE), or a microarray. All of the following methods are applicable to each of the embodiments described herein for diagnosing salivary gland neoplasms. A first example of such methods is miRNA quantitation by RT-PCR using stem-loop primers for reverse transcription followed by real-time quantitative PCR using a TaqMan® probe. In this method, stem-loop reverse transcription (RT) primers are annealed to the miRNA targets and extended using reverse transcriptase. Generation of the cDNA is followed by real-time PCR with a miRNA- specific forward primer, a TaqMan probe, and a reverse primer. Quantities of the targeted miRNAs are estimated based on measurement of CT values. These methods are known in the art and described, for example, in publications and gene expression assay product bulletins of Applied Biosystems, Foster City, CA. Primers for reverse transcriptase-mediated cDNA synthesis may be provided by the provision of a shared sequence to all miRNA sequences such as, for example, a poly(A)-tail by ligation or through action of a Terminal Transferase, followed by annealing of an adapter-oligo(dT) primer. Further methods comprise the use of a stem-loop primer, and/or the use of a miRNA-specific primer. The quantitative amplification of the RNA sequences, preferably by real-time PCR, preferably comprises a universal primer and a miRNA- specific primer.

The primers used for detection, cDNA synthesis and/or amplification preferably comprise RNA nucleotides, DNA nucleotides or modified nucleotides such as Locked Nucleic Acid (LNA) nucleotides, Peptide Nucleic Acid (PNA) nucleotides, and/or 2'-O-alkyl modifications, 2'-fluoro modifications, 4'-thio modifications, a phosphorotioate linkage, a morpholino linkage, a phosphonocarboxylate linkage. In a preferred embodiment, the length of a primer, preferably a miRNA- specific primer, is identical to the length of the specific miRNA. In a further preferred embodiment, the length of the miRNA- specific primer is shorter than the length of the miRNA, for example 14 nucleotides, 15 nucleotides, 16 nucleotides, 17 nucleotides, 18 nucleotides, 19 nucleotides, 20 nucleotides, 21 nucleotides, 22 nucleotides, or 23 nucleotides, depending on the length of the specific miRNA. The sequence of a primer, preferably a miRNA- specific primer, preferably comprises one or two mismatches compared to the sequence of the miRNA or the adapter sequence that is added to the miRNA, more preferably is identical to the sequence of the miRNA.

Another example of an miRNA quantitation method for use in the embodiments of the invention is SYBR Green detection method using locked nucleic acid (LNA)-based primers (miRCURY™ LNA microRNA PCR system, Applied Biosystems, Foster City, CA; See M. Lunn, et al. Nature Methods, February 2008) or Exiqon's microRNA qPCR system. In this method, miRNAs are reverse transcribed from total RNA in a sample using miRNA- specific RT primers, and the reverse- transcribed miRNAs are amplified using an LNA-enhanced PCR primer anchored in the miRNA sequence together and a universal PCR primer. Amplified miRNAs are quantitated by detection of fluorescence in the SYBR Green assay.

In yet another embodiment, the measuring may be performed by hybridization on a chip or microarray having the miRNAs of the AHF panel according to the invention as features thereon. The quantity of an miRNA in the sample being tested is typically determined by measurement of the fluorescence intensity of hybridization to the corresponding feature.

Another assay that can be used in the embodiments of the invention for quantitation of the relevant miRNAs is the Luminex® branched DNA (bDNA) assay (Panomics, Fremont, CA). This is a high-throughput multiplex bead-based assay based on the xMAP® technology of Luminex Corporation. Specific miRNAs are captured on their respective beads by hybridization with a capture probe, followed by sequential hybridization of pre- amplifier, amplifier and biotinylated label probes. Binding with streptavidin-conjugated phycoerythrin and analysis of individual beads for level of fluorescence quantifies the amount of miRNA captured by the bead. This assay is described in the Luminex product bulletins published by Panomics.

In still a further embodiment, a method of the invention comprises the use of a next-generation sequencing (NGS) platform for small RNA sequences. (NGS) is gaining popularity and has successfully been used to characterize miRNA profiles in various tissues as well as bio-fluids including blood and cerebral spinal fluid. See for example Wu et al. Clinica Chimica Acta 2012, 413(13-14):1058-1065; Burgos et al. RNA 2013, 19(5):712-722.

Methods and means for detection of the circulating miRNA signature of the present invention are preferably provided as a kit. Accordingly, the invention also provides a kit comprising a plurality of primers and/or probes specific for determining expression levels at least four, preferably at least five of hsa-miR-449b, hsa-miR-374, hsa-miR-411, hsa-miR-599, hsa-miR-1285, hsa-miR-324-5p and hsa-miR-449a. Preferred miRNAs and combinations thereof are discussed herein above. In one aspect, the kit comprises primers and/or probes designed to detect each of hsa-miR-449b, hsa-miR-374, hsa-miR-411, hsa-miR-599, hsa-miR-1285, hsa-miR-324-5p and hsa-miR-449a. The kit may further comprise primers and/or probes designed to detect at least one of hsa-miR-449b, hsa-miR-374, hsa-miR-411, hsa-miR-599, hsa-miR-1285, hsa-miR-324-5p and hsa-miR-449a Said kit preferably comprises one or more reagents for RT- PCR or reverse transcription RT-PCR. For example, the kit comprises a set of primers, preferably at least one specific set of primers, enzymes such as a RNA-dependent DNA polymerase and/or a DNA- dependent DNA polymerase, and at least one buffer for performing the reaction or reactions. The kit components may be provided as dried material, for example after lyophilisation, or as a liquid. In one embodiment, the kit contains reagent(s) for small RNA isolation from a (plasma) sample, such as one or more of lysis buffer, binding columns, wash and elution buffers.

A still further aspect relates to a biochip (e.g. microarray) comprising a panel of isolated nucleic acids, wherein said panel comprises at least four of hsa-miR-449b, hsa-miR-374, hsa-miR-411, hsa-miR-599, hsa-miR-1285, hsa-miR-324-5p and hsa-miR-449a, or a complement thereof. The biochip preferably comprises each of hsa-miR-449b, hsa-miR-374, hsa-miR-411, hsa-miR-599, hsa-miR-1285, hsa-miR-324-5p and hsa-miR-449a, or its complement. It could further be advantageous to include other miRNAs, such as hsa-miR-519b-3p, hsa-miR-367 and/or hsa-miR-224 to further strengthen the diagnosis.

Any of the diagnostic/predictive methods described herein above may be implemented on tangible computer-readable medium comprising computer-readable code that, when executed by a computer, causes the computer to perform one or more operations. Provided herein is a tangible, computer-readable medium comprising computer-readable code that, when executed by a computer, causes the computer to perform operations comprising: a) receiving information corresponding to the level of expression of at least four miRNA's selected from the group consisting of hsa-miR-449b, hsa-miR-374, hsa-miR-411, hsa-miR-599, hsa-miR-1285, hsa-miR-324-5p and hsa-miR-449a, in a sample of a patient having a salivary gland tumor; and b) determining a difference value in the expression level using the information corresponding to the expression level in the sample compared to a control or reference level. In some embodiments, there is a tangible computer-readable medium comprising computer-readable code that, when executed by a computer, causes the computer to perform operations comprising: a) receiving information corresponding to a level of expression in a biological sample from a subject, at least four of hsa-miR-449b, hsa-miR-374, hsa-miR-411, hsa-miR-599, hsa-miR-1285, hsa-miR-324-5p and hsa-miR-449a; and b) determining a biomarker panel value using information corresponding to the at least four biomarker miRNAs and information corresponding to the level of expression of a comparative microRNA panel from a person with a benign tumor, a person with a malignant tumor and a control person having no salivary gland tumor, the biomarker panel value being indicative of the type of salivary gland tumor. In additional embodiments the medium further comprises computer-readable code that, when executed by a computer, causes the computer to perform one or more additional operations comprising: sending information corresponding to the biomarker value to a tangible data storage device. In even further embodiments, the tangible computer-readable medium has computer-readable code that, when executed by a computer, causes the computer to perform operations further comprising: c) calculating a diagnostic score for the biological sample, wherein the diagnostic score is indicative of the benign or malignant nature of the salivary gland tumor. It is contemplated that any of the methods described above may be implemented with tangible computer readable medium that has computer readable code, that when executed by a computer, causes the computer to perform operations related to the measuring, comparing, and/or calculating a diagnostic score related to the nature of the salivary gland tumor.

In yet another embodiment, the invention relates to a system for drug discovery, comprising detecting the level of one or more of the miRNAs of the set of biomarkers of the present invention. For example, provided is a method for identifying a candidate drug e.g. for the treatment of a salivary gland tumor, by evaluating its capacity to affect the pathway(s) responsible for modulating the level of one or more of these miRNAs. The screening may involve detecting at least one miRNA selected from the group consisting of hsa-miR-449b, hsa-miR-374, hsa-miR-411, hsa-miR-599, hsa-miR-1285, hsa-miR-324-5p and hsa-miR-449a. Preferably, the screening involves measuring the level of multiple miRNAs, for example at least three miRNAs, preferably at least four, or five, but most preferably all of the biomarkers selected from the group consisting of hsa-miR-449b, hsa-miR-374, hsa-miR-411, hsa-miR-599, hsa-miR-1285, hsa-miR-324-5p and hsa-miR-449a. More preferably the screening comprises at least one of the group of hsa-miR-374, hsa-miR-411, hsa-miR-1285 and hsa-miR-324-5p, more preferably two, three or all four of this group
Suitable screening systems include cell-based assays, for example cells cultured from salivary glands as described by Tran, S.D. et al., 2005, Tissue Eng. 11:172-181 or Chan, Y.H. et al., 2011, J. Cell Physiol. 226:3076-3085, and especially cells from salivary gland tumors, such as described by Yoshida, M. et al., 1980, Br. J. Cancer 41:636-639 and cells from cell line TCP-1012 (ATCC).

The invention thus also relates to a method for screening a pharmaceutically active compound for the treatment and/or prophylaxis of a salivary gland tumor, wherein the method comprises the steps of (a) providing a cell comprising an expression vector comprising at least miRNA sequence selected from the group consisting of hsa-miR-449b, hsa-miR-374, hsa-miR-411, hsa-miR-599, hsa-miR-1285, hsa-miR-324-5p and hsa-miR-449a, more preferably at least one of the group of hsa-miR-374, hsa-miR-411, hsa-miR-1285 and hsa-miR-324-5p; (b) bringing a candidate for a pharmaceutically active compound into contact with the cell, (c) determining the effect of the candidate on the expression of said at least one miRNA sequence, wherein a change in the expression of said at least one miRNA sequence might indicate a pharmaceutically active compound. Preferably, the screening method comprises determining the effect on at least three, preferably at least all four miRNA sequences selected from the group of hsa-miR-374, hsa-miR-411, hsa-miR-1285 and hsa-miR-324-5p.

Alternatively, such a method comprises the steps of: (a) providing a culture from a cell line derived from a human or animal salivary gland tumor, (b) optionally measuring the expression of any of the miRNAs of the set of biomarkers according to the present invention; (c) bringing a candidate for a pharmaceutically active compound into contact with the cell; and (d) determining the effect of the candidate on the expression of said at least one miRNA sequence, wherein a change in the expression of said at least one miRNA sequence might indicate a pharmaceutically active compound. It is further possible, of course, to prolong the treatment time with the pharmaceutical compound, by providing the compound to the cell culture more than once and to extend the number of assays for the expression of the set of biomarkers, to see if there are changes over time.

Also part of the invention is a method for monitoring treatment of a salivary gland tumor. It is submitted that the amount of expression of at least one of the biomarkers provided herein, but especially the biomarkers that are associated with a malignant salivary gland neoplasm is indicative for the severeness of the disease and a decrease in the level of such a biomarker that is specific for a malignant tumor would be indicative of a success of the treatment. Accordingly, the present invention comprises a method for monitoring treatment of a malignant salivary gland tumor by periodically checking before, during and/or after treatment of the expression of at least one miRNA sequence selected from the group consisting of hsa-miR-449b, hsa-miR-374, hsa-miR-411, hsa-miR-599, hsa-miR-1285, hsa-miR-324-5p and hsa-miR-449a, more preferably at least one of the group of hsa-miR-374, hsa-miR-411, hsa-miR-1285 and hsa-miR-324-5p.

### Examples

### Example 1. Patient selection and salivary miRNA profiling

Whole saliva samples from patients with malignant (*n*=38) or benign parotid gland tumors (*n*=29) were obtained from the Salivary Gland Tumor Biorepository at the MD Anderson Clinic (Houston, TX). Samples were immediately stored at -80°C until ready for use. Before the samples were used, they were defrosted on ice and centrifuged for 10 min at 5,000 rpm at 4°C. The cell-free supernatant was collected from the pellet and used immediately in the next step.
In the discovery phase, samples of whole saliva from 10 patients with benign parotid gland tumor were matched (gender and ethnicity) and analyzed. Both benign and malignant groups consisted of 7 men and 3 women. The mean age was 53 (33-82) and 60 (49-74) years of the benign and malignant group, respectively. Both groups consisted of the same ethnic background (1 Hispanic, 1 Black, 8 Caucasians). Smoking/drinking habits or clinicopathologic data were neither recorded nor provided by the SGTB and were irretrievable. Patient's characteristics are given in Table 1.
An independent sample set was used for the validation study (19 benign and 28 malignant).

**Table 1. Clinicopathological data of patients with a benign parotid or malignant parotid gland tumor.**

| **Clinicopathological data** | | |
|---|---|---|
| | *Benign* | *Malignant* |
| *Mean age* | 53 | 60 |
| *(range)* | (33-82) | (49-74) |
| | | |
| *sex (male*/*female)* | 6/4 | 6/4 |
| *Ethnicity* | | |
| Hispanic | 1 | 1 |
| Caucasian | 8 | 8 |
| Black | 1 | 1 |
| | | |
| *Neoplasm subtypes* | | |
| Pleomorphic adenoma | 6 | |
| Warthins tumor | 3 | |
| Oncocytoma | 1 | |
| Neuroendocine carcinoma | | 1 |
| Oncocytic carcinoma | | 1 |
| Acinic cell carcinoma | | 1 |
| Mucoepidermoid carcinoma | | 1 |
| Salivary duct carcinoma | | 2 |
| Myoepithelial carcinoma | | 1 |
| Carcinoma ex-pleomorphic adenoma | | 1 |
| Cystadenocarcinoma | | 1 |
| Adenoid cystic carcinoma | | 1 |

Total RNA was isolated from 300 µL saliva supernatant using RNA extraction kits (Ambion mirVana Paris kit). DNase I treatment (DNase I, Qiagen) was used to remove contaminating DNA furing RNA extraction. The concentration of total RNA was measured using RiboGreen assay. Extracted RNA (1 - 10 ng) was reverse transcribed and preamplified using the TaqMan MicroRNA reverse Transcription Kit, TaqMan PreAmp Master Mix, and Megaplex Primers (applied Biosystems). The preamplification product was not diluted before conducting miRNA quantification. For the profiling of 750 miRNAs a total volume of 105 µL was loaded into each well of the TaqMan Human MicroRNA Cards (Applied Biosystems), which were spun and run on the Applied Biosystems 7900HT Fast Real-Time PCR instrument containing a special card holder (Applied Biosystems). Using default TaqMan low density array setting and 6-carboxyfluorescein as a reporter, the quantitative real-time PCR (qRT-PCR) reaction was run at 95°C for 10 min to activate the enzyme and was then followed with 40 cycles at 95°C for 15 sec and 60°C for 60 sec.
The cycle threshold (Ct) value is defined as the cycle number in the fluorescence emission, which exceeds that of a fixed threshold. A Cₜ of 15 to 30 was considered high expression and a Ct of 35 or higher is considered low expression. A Ct value of more than 40 was considered as undetectable miRNA. For miRNA quantitative PCR (qPCR) experiments, U6 snRNA was used as the reference gene. We calculated ΔCₜ by substracting the Ct value of the reference gene (RNA polymerase III transcribed U6 snRNA) from the Ct value of each candidate biomarker. Data normalization was conducted using RQ manager 1.2.1 and Data Assist v3.0 from Applied Biosystems. The qPCR based gene expression values between the 2 groups were statistically compared.

### Example 2. Statistical analysis

Our statistical analysis is based on application of the adapted version of the elastic net algorithm (14) which is specifically tailored for discriminating among benign and malignant groups in the collected dataset. It consists of learning and stability selection modules and allows for robust and reliable identification of miRNA biomarkers. We provide a detailed description of the approach in the supplementary material and a flow diagram of the study (Fig. 1).
The elastic net method proposed in (14) is a hybrid combination of the LASSO method (16) and ridge regression (17). A combination of these methods is used to provide the advantages that each method holds.

For example, LASSO cannot utilize correlation between coefficients because it tends to randomly select only one feature from a correlated group. Situations may occur in which a single feature is useless, but when combined with other features, they may provide better predictive performance (23). In our problem, we expected group-based correlations among the miRNAs to occur and, thus, between their corresponding regression coefficients. In such situations, the predictive performance of ridge regression tends to outperform that of LASSO (3). On the other hand, the ridge regression was unable to shrink coefficients to zero. The LASSO method was able to shrink coefficients to zero by imposing an L1-penalty on the regression coefficients. This allowed for an easily interpretable model by viewing any nonzero coefficients as the predictors that had the strongest predictive power for a posed problem. A comparison of the predictive performances of the LASSO, ridge regression and other methods was made by Tibshirani (24) and Fu (25). It has been noted that when conducting biomarker identification, due to the possibility of coefficients shrinking to zero, the LASSO appeared to be a much more sensible choice.
To combine the feature sparsity of LASSO and feature smoothness of ridge regression, we used an adapted elastic net technique which exploited the ridge regression tendency of shrinking coefficients to small values for correlated trending towards each other, while retaining the feature selection property of reducing coefficients to exact zero values provided by LASSO. This encouraged a 'grouping effect', providing subsets of correlated features (14). This effect was also useful when analyzing miRNA datasets due to our aim to detect group-based miRNAs interactions that allowed us to study them as a mechanistic system rather than as separate factors affecting tumorigenesis. The elastic net was often used for feature selection within MS data (26, 27). The main motivation for choosing the elastic net method was to select the most important features, while retaining the correlation between features.
The biomarkers were identified by elastic net regression as the feature selection model. This model was parameterized by a tuning constant λ, modulating the emphasis of either L1 or L2 regularization. Such parameters in combination with multiplication factor α controlled the number of features selected (14). Stronger L1 regularization results in a lower false discovery rate, but may miss real-associated features; while stronger L2 regularization detects more real-associated features, but also admits more false positives. Thus, choosing the right value of the regularization parameter λ becomes critical, and we have to make sure that a model consistently selects truly relevant features. To minimize influence of such a critical choice on the model performance, we use stability selection procedure.. Biomarker stability (17) is reflected in the frequency that a particular biomarker was identified in multiple simulations on a re-randomized dataset. This measure is especially relevant for small to medium sized data collections (as this study where the number of patients diagnosed with a malignant form of disease is limited).
Stability selection (28) is a technique designed to improve the performance of a feature selection algorithm based on the sub-sampling of examples. The general idea of stability selection is to select only those features that are consistently selected throughout multiple runs of a feature selection model. This is done by running elastic net many times on randomly subsampled data and choosing those variables that are selected most frequently across all sample partitions. In our task, we run elastic net on multiple random sub-sampled datasets containing both benign and malignant tumor samples. The performance measure used for a binary classification task is a Receiver Operating Characteristics Area Under Curve (ROC AUC). The ROC can be understood as a plot of the probability of correctly classifying the positive samples (benign group) against the rate of incorrectly classifying true negative samples (malignant group). Thus, the AUC score of an ROC curve can be considered a measure of the predictive accuracy of the model.
To avoid over-fitting, we used a 5-fold stratified cross-validation procedure over the train partition of the data (80%) while the remaining 20% was used as the testing dataset. Parameters to be selected are ratio between L1 and L2 norms, L1-2-ratio and regularization parameter multiplication coefficient α. Stability selection was performed by randomly subsampling 80% of the data 200 times. During stability selection procedure, all features having non-zero weighting coefficient were counted.

Stability selection procedure conducted on the miRNA expression profiles allowed us to select the most robust biomarker miRNAs. The stability coefficient levels of the miRNAs starts to level off after 0.44, which results in a plateau around 0.3. MiRNAs having stability coefficient levels of 0.44 and higher were considered important and were therefore selected. These biomarkers, as well as their stability coefficients, are presented in Table 2. We observed miRNA hsa-miR-449b to have a high stability coefficient of 0.88. Other selected miRNAs with stability coefficients (0.6 ÷ 0.4) are hsa-miR-374, hsa-miR-411, hsa-miR-599, hsa-miR-1285, hsa-miR-324-5p, and hsa-miR-449a. The ROC curve with the best prediction was accomplished by combining all 7 selected miRNAs, resulting in an AUC classification performance of 0.92 (specificity 86%; sensitivity 91%) (Fig. 2A). This result was statistically significant (P < 0.01) by application of randomization test (Supplementary Fig. S2), and it demonstrates that the most stable biomarkers are also highly predictive in terms of classification output.

To visualize how the values of the selected biomarkers differ per group, we constructed pie charts based on the data from both benign and malignant tumors. For each of the seven selected miRNAs, we calculated the sum of ΔCt for malignant and benign tumor groups (Fig. 2D). In the benign group, the sum of ΔCt of hsa-miR-449b, hsa-miR-449a and hsa-miR-599 was lower compared to the malignant group. The sum of ΔCt of hsa-miR-324-5p, hsa-miR-374, hsa-miR-411 and hsa-miR-1285 was higher in the benign group compared to the malignant group.

**Table 2. Stability coefficients and average ΔCₜ for the discovered miRNAs**

| miRNA | Stability coefficient | Average ΔCₜ Benign | Average ΔCₜ Malignant | Expression level higher in |
|---|---|---|---|---|
| hsa-miR-449b | 0.88 | 15.1 | 18.5 | B |
| hsa-miR-374 | 0.65 | 7.2 | 0 | M |
| hsa-miR-411 | 0.63 | 19.5 | 18.5 | M |
| hsa-miR-599 | 0.57 | 16.3 | 21 | B |
| hsa-miR-1285 | 0.56 | 16.4 | 9.6 | M |
| hsa-miR-324-5p | 0.46 | 16.5 | 11.8 | M |
| hsa-miR-449a | 0.44 | 14 | 15.9 | B |
| hsa-miR-519b-3p | 0.36 | 4.8 | 12.3 | B |
| hsa-miR-367 | 0.35 | 12 | 15.1 | B |
| hsa-miR-224 | 0.34 | 8.7 | 10.7 | B |

| | | | | |
|---|---|---|---|---|
| B= benign; M= malignant. | | | | |

Besides ΔCₜ values, estimated Elastic Net coefficients also play key a role in classification of tumor samples. To visualize the joint influence of the Elastic Net coefficients and ΔCₜ, we plot the product of the Elastic Net coefficient and ΔCₜ for both malignant and benign tumors (Fig. 2B and 2C). The overall sum of all products per each sample results in a classification prediction, resulting in two different patterns. While, in the malignant tumor group the overall sum of the product was in a positive direction, the opposite is true for the benign tumor group in which the overall sum of the product was in the negative direction.

While solving a penalized regression problem, we assume that due to the biological nature of the problem, there is a correlation between miRNA expression value matrix and classification label vector. This assumption can be tested by conducting modeling on the dataset with reshuffled class labels while keeping the corresponding miRNA expression value indices fixed. Then the underlying statistical relationship will be disrupted. Such a procedure is often used to test the statistical validity of results and is known as randomization test (29).

To check statistical significance of the model results, we define ROC AUC score as a test statistic score. The null-hypothesis is that the order of labels for the classification test does not matter, meaning that the model will give good ROC AUC score even if the order of labels is shuffled. A histogram for the null-hypothesis distribution can be generated by repeating the randomization multiple times and calculating corresponding AUC scores. This histogram can be used as a statistical significance test of the model reflecting the relationship between miRNA and classification labels. However, in practice, any shuffling of the labels still leaves some correlation between the shuffled and original data which needs to be taken in account. Therefore, we need to define a critical value derived from the null-distribution as the value exceeded by a certain percentage of noise values (usually either 5% or 1%). The statistics score obtained for the original data, in our case true AUC, is compared with the critical value.
In our case, we state the null-hypothesis that the order of the labels for the classification test does not matter, and the model will give good AUC score even if the order of labels is shuffled. To reject null hypothesis, we randomly shuffle labels 10000 times and retrain Elastic Net model on the labels using 3-fold cross-validation on 100% data to define parameters and apply these parameters on the same dataset. The result of the randomization test is depicted as histogram plot of AUC scores on Fig. 4.
The average AUC for true non-shuffled labels on 3-fold cross validation for selected seven biomarkers is around 0.93 and is shown in Fig. 4 as the red vertical line. As we see, most of the results are less than 0.93. Moreover, most of the results of the randomized model are around 0.5, which refers to completely random classification results. To accept or reject null-hypothesis, we calculate p-value as a ratio between the values of AUC ≥ AUCtrue and values of AUC < AUCtrue. The P-value for 10000 shuffles is 0.0097 < 0.01, thus assuming 1% significance level, we can reject null-hypothesis.

Since we have rejected the null-hypothesis, we accept the fact that the order of the labels is important, and we may not incorrectly label benign tumors as malignant ones and other way around. This result suggests that dataset correctly reflects differences between two types of tumors, and the Elastic Net model selects valid biomarkers.

### References

1. Seifert G, Brocheriou C, Cardesa A, Eveson JW. WHO International Histological Classification of Tumours. Tentative Histological Classification of Salivary Gland Tumours. Pathol Res Pract 1990;186:555-81.
2. Iorio MV, Croce CM. MicroRNAs in cancer: small molecules with a huge impact. J Clin Oncol 2009;27:5848-56.
3. Chen PS, Su JL, Hung MC. Dysregulation of MicroRNAs in cancer. J Biomed Sci 2012;19:90.
4. Ueda T, Volinia S, Okumura H, Shimizu M, Taccioli C, Rossi S, et al. Relation between microRNA expression and progression and prognosis of gastric cancer: a microRNA expression analysis. Lancet Oncol 2010;11:136-46.
5. Mitchell PS, Parkin RK, Kroh EM, Fritz BR, Wyman SK, Pogosova-Agadjanyan EL, et al. Circulating microRNAs as stable blood-based markers for cancer detection. Proc Natl Acad Sci USA 2008;105:10513-8.
6. Matse JH, Yoshizawa J, Wang X, Elashoff D, Bolscher JGM, Veerman ECI, et al. Discovery and pre-validation of salivary extracellular microRNA biomarkers panel for the non-invasive detection of benign and malignant parotid gland tumors. Clin Cancer Res 2013;9:3032-8.
7. Brinkmann O, Wong DT. Salivary transcriptome biomarkers in oral squamous cell cancer detection. Adv Clin Chem 2011;55:21-34.
8. Li Y, Elashoff D, Oh M, Sinha U, St John MA, Zhou X, et al. Serum circulating human mRNA profiling and its utility for oral cancer detection. J Clin Oncol 2006;24:1754-60.
9. Zhang L, Xiao H, Karlan S, Zhou H, Gross J, Elashoff D, et al. Discovery and preclinical validation of salivary transcriptomic and proteomic biomarkers for the non-invasive detection of breast cancer. PLoS One 2010;5:e15573.
10. Hu S, Arellano M, Boontheung P, Wang J, Zhou H, Jiang J, et al. Salivary proteomics for oral cancer biomarker discovery. Clin Cancer Res 2008;14:6246-52.
11. Li Y, St John MA, Zhou X, Kim Y, Sinha U, Jordan RC, et al. Salivary transcriptome diagnostics for oral cancer detection. Clin Cancer Res 2004;10:8442-50.
12. Imangaliyev S, Keijser B, Crielaard W, Tsivtsivadze E. Online Semi-supervised Learning: Algorithm and Application in Metagenomics. IEEE BIBM 2014.
13. Hastie T, Tibshirani R, and Friedman J. The elements of statistical learning: data mining, inference and prediction. 2nd edition (2009), Springer.
14. Zou H, Hastie T. Regularization and variable selection via the Elastic Net. J. Roy. Statist. Soc. Ser. B 2005;v67
15. Cornelisse LN, Tsivtsivadze E, Meijer M, Dijkstra TMD, Heskes T, Verhage M. Molecular machines in the synapse: Overlapping protein sets control distinct steps in neurosecretion. PloS Computation Biology 2012;8:e1002450.
16. Hoerl A, Kennard RW. Ridge Regression: Applications to Nonorthogonal Problems. Technometrics 1970;12:69-82.
17. Meinshausen N, Bühlmann P. Stability selection. J. Roy. Statist. Soc. Ser. B 2010;72:417-73.
18. Wang XW, Zhan Q, Coursen JD, Khan MA, Kontny HU, Yu L, et al. GADD45 induction of a G2/M cell cycle checkpoint. Proc Natl Acad Sci USA 1999;96:3706-11.
19. Bueno MJ, Malumbres M. MicroRNAs and the cell cycle. Biochim Biophys Acta 2011;1812:592-601.
20. Fan W, Richter G, Cereseto A, Beadling C, Smith KA. Cytokine response gene 6 induces p21 and regulates both cell growth and arrest. Oncogene 1999;18:6573-82.
21. Tian S, Huang S, Wu S, Guo W, Li J, He X. MicroRNA-1285 inhibits the expression of p53 by directly targeting its 3' untranslated region. Biochem Biophys Res Commun 2010;396:435-9.
22. Yang X, Feng M, Jiang X, Wu Z, Li Z, Aau M, et al. miR-449a and miR-449b are direct transcriptional targets of E2F1 and negatively regulate pRb-E2F1 activity through a feedback loop by targeting CDK6 and CDC25A. Genes Dev 2009;23:2388-93.
23. Guyon I, Elisseeff A. An introduction to variable and feature selection. JMLR. 2003; 3: 1157-1182
24. Tibshirani R. Regression shrinkage and selection via the lasso. J. R. Stat. Soc. Series B: 1996; 58: 267-288.
25. Fu WJ. Penalized Regressions: The Bridge Versus the Lasso Journal of Computational and Graphical Statistics. 1998; 7: 397-416
26. Saeys Y, Inza I. & Larranaga, P. (). A review of feature selection techniques in bioinformatics. Bioinformatics, 2007; 23: 2507-2517.
27. Zhang FZ, Hong D. Elastic net-based framework for imaging mass spectrometry data biomarker selection and classification. Stat Med. 2011; 30: 753-68.
28. Meinshausen N. and Bühlmann P. Stability selection. J. R. Stat. Soc.: Series B. 2010; 72: 417-473.
29. Fisher RA. The design of experiments. Edinburgh: Oliver and Boyd. 1935.
30. Lizé M, Pilarski S, Dobbelstein M. E2F1-inducible microRNA 449a/b suppresses cell proliferation and promotes apoptosis. Cell Death Differ 2010;17:452-8.
31. Ye W, Xue J, Zhang Q, Li F, Zhang W, Chen H, et al. MiR-449a functions as a tumor suppressor in endometrial cancer by targeting CDC25A. Oncol Rep 2014;32:1193-9.

## Claims

1. A set of biomarkers for the diagnosis of salivary gland neoplasms wherein said set consists of at least four markers selected from the group of hsa-miR-449b, hsa-miR-374, hsa-miR-411, hsa-miR-599, hsa-miR-1285, hsa-miR-324-5p and hsa-miR-449a.

2. A set according to claim 1, wherein said set consists of at least five, more preferably at least six and most preferably at least seven of said biomarkers.

3. A set according to claim 1 or 2, wherein the presence of hsa-miR-449b, hsa-miR-599, and/or hsa-miR-449a is indicative for the presence of a benign salivary gland neoplasm, while the presence of hsa-miR-324-5p, hsa-miR-1285, hsa-miR-411 and/or hsa-miR-374 is indicative of a malignant salivary gland neoplasm.

4. A kit for the diagnosis of salivary gland neoplasms comprising means for measuring the level of the set of biomarkers as defined in one or more of the previous claims in a sample of a person and instructions for use.

5. A method for the diagnosis of the benign or malignant status of salivary gland neoplasms comprising
a) taking a sample from a person;
b) determining the amount of any of the sets of biomarkers as defined in any of claims 1 - 3; and
c) on basis of these results classifying the salivary gland neoplasm as benign or malignant.

6. A method according to claim 5, wherein the sample is a saliva sample.

7. A method for screening a pharmaceutically active compound for the treatment and/or prophylaxis of a salivary gland tumor, wherein the method comprises the steps of
(a) providing a cell comprising an expression vector comprising at least a miRNA sequence selected from the group consisting of hsa-miR-449b, hsa-miR-374, hsa-miR-411, hsa-miR-599, hsa-miR-1285, hsa-miR-324-5p and hsa-miR-449a;
(b) bringing a candidate for a pharmaceutically active compound into contact with the cell,
(c) determining the effect of the candidate on the expression of said at least one miRNA sequence, wherein a change in the expression of said at least one miRNA sequence indicates a pharmaceutically active compound.

8. A method for screening a pharmaceutically active compound for the treatment and/or prophylaxis of a salivary gland tumor, wherein the method comprises the steps of
(a) providing a cell from a salivary gland tumor celline;
(b) bringing a candidate for a pharmaceutically active compound into contact with the cell,
(c) determining the effect of the candidate on the expression of at least one miRNA sequence selected from the group consisting of hsa-miR-449b, hsa-miR-374, hsa-miR-411, hsa-miR-599, hsa-miR-1285, hsa-miR-324-5p and hsa-miR-449a, more specifically at least one of the group of hsa-miR-374, hsa-miR-411, hsa-miR-1285 and hsa-miR-324-5p wherein a change in the expression of said at least one miRNA sequence indicates a pharmaceutically active compound.

9. Method for monitoring treatment of a malignant salivary gland tumor by periodically checking before, during and/or after treatment of the expression of at least one miRNA sequence selected from the group consisting of hsa-miR-449b, hsa-miR-374, hsa-miR-411, hsa-miR-599, hsa-miR-1285, hsa-miR-324-5p and hsa-miR-449a, more preferably at least one of the group of hsa-miR-374, hsa-miR-411, hsa-miR-1285 and hsa-miR-324-5p.
